# EUROPEAN PATENT APPLICATION

(11) **EP 2 283 859 A2**
(43) Date of publication of application: **16.02.2011**
(21) Application number: 10176280.5
(22) Date of filing: 26.10.2005
(51) Int. Cl.: A61K 39/215, A61K 48/00, C07K 14/165, C07K 16/10, C12N 7/06, C12N 7/08, C12N 15/50, G01N 33/53, G01N 33/569

(54) **Pantropic canine coronavirus**

(62) Divisional of application: 05023371.7
(71) Applicant: Buonavoglia, Canio, 70016 Noicattaro (BA) (IT)
(72) Inventor: Buonavoglia, Canio, 70016 Noicattaro (BA) (IT); Decaro, Nicola, 70010 Valenzano (BA) (IT); Martella, Vito, 70010 Valenzano (BA) (IT); Elia, Gabriella, 70010 Valenzano (BA) (IT); Campolo, Marco, 70010 Valenzano (BA) (IT); Desario, Costantina, 70010 Valenzano (BA) (IT); Tempesta, Maria, 70010 Valenzano (BA) (IT)
(74) Representative: Banfi, Paolo

(57) **Abstract**

The invention provides a pantropic, highly pathogenic variant of canine coronavirus type II that is responsible for acute, fatal disease in dogs, polynucleotide and protein sequences thereof and their use in the prophylaxis, therapy and diagnosis of canine infections.

SEQUENCE LISTING

<110>  Buonavoglia, Canio

<120>  pantropic canine coronavirus

<130>  2378EURDIV

<160>  19

<170>  PatentIn version 3.3

<210>  1
<211>  8745
<212>  DNA
<213>  Unknown

<220>
<223>  canine coronavirus

<400>  1
ggtaagttgc tcattagaaa taatggcaag ctactaaact ttggtaatca tttagttaat      60

gtgccatgat tgtgatctta acttgcgtct tattgttgtg ctcgtaccat actgttgcga     120

gtacgacaaa taatgattgt agacaagtta acgtaacaca attagatggc aacgaaaatc     180

tcattagaga ctttttgttt caaaacttta aagaagaagg aactgtagtt gttggtggct     240

attatcctac agaggtgtgg tacaactgtt ctagaacagc acctaccact gcctatgagt     300

attttaataa catacatgca ttttattttg atatggaagc catggaaaat agcactggta     360

atgcacgtgg taaaccgtta ttgtttcatg ttcatggtga gcctgttagt gctatcatat     420

atatatcggc ttatagaaat gatgtacaac acaggccact tttaaaacat gggttagtgt     480

gcataactaa aactcgcaat attgactata acagcttcac tagcagacag tggaattcca     540

tatgtacagg taatgacaga aaaattcctt tctctgtcat acccacggac aatggaacaa     600

aaatctatgg acttgagtgg aatgacgaat ttgttacagc ctatattagt ggtcattctt     660

ataattggaa catcaataat aattggttta acaatgttac actactttat tcccgctcaa     720

gtactgctac atggcaacac agtgctgcat atgtttacca aggtgtttct aacttcactt     780

attacaagtt aaataacacc aatggtttaa aaacttatga attttgtgat gattatgaat     840

attgcactgg ctacgctact aatgtctttg ctcccactgt aggaggttac atacctgatg     900

gatttagttt taacaattgg tttttgctta caaatggctc cacttttgtt agtggcagat     960

ttgtaacaaa tcaaccatta ttagttaatt gtttatggcc agtgcccagt tttggtgtgg    1020

cagcacaaga attttgtttt gaaggtgcgc agtttagtca gtgcaatggt gtgtctttaa    1080

ataacacagt ggatgttatt agattcaatc ttaattttac cgcagatgta caatctggca    1140

tgggtgctac agtgttctca ttgaatacaa caggtggtgt cattcttgaa atttcatgtt    1200

ataatgacac agtgagtgag tctagttttt acagttatgg tgaaattcca tttggcataa 1260

ctgatggacc acggtactgt tatgcacttt acaatggcac agctcttaag tatttaggaa 1320

cattaccacc tagtgtaaag gaaattgcta ttagtaagtg gggccatttt tatattaatg 1380

gttacaattt ctttagcaca tttcctattg attgtatatc ttttaattta accactggta 1440

ctagtggagc tttttggact attgcttaca catcgtacac tgaagcatta gtacaagttg 1500

aaaacacagc tattaaaaag gtgacgtatt gtaacagtca cattaataac attaaatgtt 1560

ctcaacttac tgctaatttg caaaatggtt tttatcctgt tgcttcaagt gaagttggtc 1620

ttgtcaataa gagtgttgtg ttactaccta gtttctattc acataccagt gttaatataa 1680

ctattgatct tggtatgaag cgtagtggtt atggtcaacc tatagcctca gcattaagta 1740

acatcacact accgatgcag gataataaca ccgatgtgta ttgcattcgt tctaatcaat 1800

tttcagttta cgtgcattcc acttgtaaaa gttctttatg ggacaatgtg tttaattctg 1860

actgcacaga ttttttacat gctacagctg ttataaaaac tggtacttgt cctttctcat 1920

ttgataaatt gaacaattac ctaactttta acaagttctg tttttcattg aatcctgttg 1980

gtgccaattg caaatttgat gttgctgccc gtacaagaac caatgagcag gttgttagaa 2040

gtttatatgt gatatatgaa gaaggagaca atatagtggg tgtaccgtct gataatagtg 2100

gtctgcacga tttgtcagtg ttacacttag actcctgtac agattacaat atatatggta 2160

gaactggtgt tggtattatt agacaaacta acagcacact acttagtggc ttatattaca 2220

catcactatc aggtgatttg ttaggtttta aaaatgtcac tgacggtgtt gtctattctg 2280

taacaccatg tgatgtaagt gcacaagctg ctgttattga tggtgccata gttggagcta 2340

tgacttccat taatagtgaa ctgttaggtc taactcattg gacaacaaca cctaattttt 2400

attattactc catatataat tatacaaatg cgagaactcg tggcactgca atcgacagta 2460

acgatgttga ttgtgaacct atcataacct attctaatat aggtgtttgt aaaaatggag 2520

ctttggtttt tattaacgtc acacattctg atggtgacgt tcaaccaatt agcaccggta 2580

atgtcacgat acctacaaat tttactatat ctgtgcaagt cgaatatatt caggtttaca 2640

ctacaccagt gtcaatagac tgttcaagat acgtctgcaa tggtaatcct agatgcaata 2700

aattgttaac acaatatgtc tctgcatgtc aaactattga gcaagcactt gcaatgggtg 2760

ccagacttga aaacatggaa gttgattcca tgttgtttgt ttctgaaaat gcccttaaat 2820

tggcatctgt tgaagcattc aatagtacgg aaactctaga tcctatttac aaagaatggc 2880

ctaacattgg tggttcttgg ctaggaggtt taaaagacat attgccatct cacaatagca 2940

aacgtaagta ccggtctgct atagaagatt tgctttttga taaggttgtg acatctggct 3000

taggtacagt cgatgaagat tacaaacgtt gtacgggtgg ctatgacata gctgacttag 3060

tgtgtgcaca atattacaat ggcatcatgg tgttacctgg tgtggctaat gatgacaaga 3120

tggctatgta cactgcatct cttgcaggtg gtataacatt aggtgcactt ggtggtggtg 3180

cagtgtctat accttttgca gtagcagttc aggctagact taattatgta gctttacaaa 3240

ctgacgtatt gaacaaaaac cagcagattc tggctaatgc tttcaatcaa gcgattggta 3300

acattacaca ggcatttggt aaggttaatg atgctataca tcaaacgtca aaaggtcttg 3360

ctactgttgc taaagcattg gcaaaagtgc aagatgttgt taacacacaa gggcaagctt 3420

taagccacct aacagtacaa ttgcaaaata attttcaagc cattagtagt tccattagtg 3480

acatttataa caggcttgat gagttgagtg cggatgcaca agttgacagg ctgattacag 3540

gacgacttac agcacttaat gcatttgtgt ctcagacttt aaccagacaa gcagaggtta 3600

gggctagtag acaacttgct aaagacaaag ttaatgaatg cgttaggtct caatcccaaa 3660

gatttggatt ctgtggtaat ggtacacatt tgttttcact tgcaaatgca gcaccaaatg 3720

gcatgatttt ctttcacaca gtgctattac caacagctta tgaaactgtg acggcctggt 3780

caggtatttg tgcatcagat ggcgatcgca cttttggact tgttgttaaa gatgtccagc 3840

tgacgctatt tcgcaattta gatgacaaat tctatttgac acctagaact atgtatcagc 3900

ctagagttgc aactagttct gattttgttc aaattgaagg gtgtgatgtc ttgtttgtca 3960

atgcaactgt aattgagttg cctagtatta tacctgatta tatcgatatt aatcagactg 4020

ttcaagacat attagaaaat tacagaccaa attggacagt acctgaatta acacttgaca 4080

ttttcaacgc aacctactta aacctgactg gtgaaattaa tgacttagaa ttcaggtcag 4140

aaaagctaca taacaccacg gtagaacttg ctgttctcat tgacaatatt aacaatacat 4200

tagtcaatct tgaatggctc aatagaattg aaacttatgt aaaatggcct tggtatgtat 4260

ggctactaat aggcttagta gtaatatttt gcataccatt actgctattt tgctgttgta 4320

gtacaggttg ctgtgggtgc ataggttgct taggaagttg ttgtcactct atttgtagta 4380

gaagacaatt tgaaaattat gaaccaattg agaaagtgca tgtccattaa attcaaaata 4440

aatctcttaa gaactaaact tatgagtcat tacaggtctt gtatggacat tgtcaaatct 4500

attgacacat ccgtagacgc tgtacttgac gaacttgatc gtgcatactt tgctgtaact 4560

cttaaagtag agtttaagac tggtaaacta cttgtgtgta taggttttgg tgatacacct 4620

cttgaggcta aggataaagc atatgctaaa cttggtttct ctattattga agaagtcaat 4680

agtcatacag ttgtttgata ttaccttttg aaactagact tcttatcatc gaacaaacaa 4740

aacctaaagc attaagtgct acaaaacaat caaagagaga ttatagaaaa attgccattc 4800

taaattccat gagaaaatga ttggtggact ttttcttaac actctgagtt ttgtaattgt 4860

tagcaaccat gtcattgtta ataatacagc aaatgtgcat tctatacaac aagaacatgt 4920

tatagtacaa cagcatcagg ttgttagtgc tagaacacaa aattactacc cagagttcag 4980

catcgctgta ctctttgtat tatttctagc tttgtaccgt agtacaaact ttaagacgtg 5040

tgtcgatatc ttaatgttta agattgtatc aatgacactt atagggccta tgcttatagc 5100

atatggttac tacattgatg gcattgttac aacaactgtc ttagctttaa gatttgtcta 5160

cttatcatac ttttggtatg ttaatagtag gtttgaattc atcttataca atacaacgac 5220

actcatgttt gtacatggca gagctgcacc gtttatgaga agttctcaca gctctattta 5280

tgtcacattg tacggtggca taaattatat gtttgtgaat gacctcacgt tgcattttgt 5340

agaccctatg cttgtaagca tagcaatacg tggcttagct catgctgatc taactgttgt 5400

tagagcagtt gaacttctca atggtgattt tatctatgta ttttcacagg agcccgtagt 5460

cggtgtttac aatgcagcct tttctcaggc ggttctaaac gaaattgact taaaagaaga 5520

agaagaagac catacctatg acgttcccta gggcattgac tgtcatagat gacaatggaa 5580

tggtcattag tatcattttc tggttcctgt tgataattat attgatatta ctttcaatag 5640

cattgctaaa tataattaag ctatgcatgg tatgttgtaa cttaggaaga acagttatta 5700

ttgttccagc gcaacatgcc tatgatgcct ataagaattt tatgcgaatt aaagcatata 5760

accccgacga agcactcctt gtttgaacta aacaaaatga agattttgtt agtattagcg 5820

tgtgcaattg catgcgcatg tggtgaacgt tattgcgcta tgaaatctga ctcagatact 5880

tcgtgtcgca atggtaccac tactgattgc gaatcatgct tcaacggagg tgatcttatt 5940

tggtatcttg caaactggaa cttcagctgg tctgtaatat tgatcgtttt tataacggtt 6000

ttacaatatg gtagacctca atttagctgg ttcgtgtatg gcattaaaat gcttattatg 6060

tggctactat ggcccattgt tctggctctt acgattttta atgcatactc ggaatacgaa 6120

gtttccagat atgtaatgtt cggctttagt gttgcaggtg caattgttac atttatactt 6180

tggattatgt attttgttag atccattcag ttatacagaa ggactaagtc ttggtggtct 6240

ttcaaccctg aaactaacgc aattctttgc gttagtgcat taggaaggag ctatgtgctt 6300

cctcttgaag gtgtgccaac tggtgtcact ttaacattgc tctcagggaa tttgtacgct 6360

gaagggttca aaattgcagg tggtatgaac atcgacaatt tgccaaagta cgtaatggtt 6420

gcattaccta gcaggaccat tgtctacaca cttgttggta agaaattgaa agcaagtagt 6480

gcaacaggat gggcttacta tgtaaaatct aaagctggtg attactcaac agatgcacga 6540

actgacaatt tgagtgagca agaaaaatta ttacatatgg tataactaaa cttctaaatg 6600

gccaaccagg gacaacgcgt tagctgggga gatgaatcta ccaaaaagcg tggtcgttcc 6660

aattctcgtg gccggaagaa taatactata cctctttcat tcttcaaccc cattaccctc 6720

caacaaggtt caaaattttg gaacttatgt ccgagagact ttgtacccaa aggaataggt 6780

aacaaggatc aacagattgg ttattggaat agacaaactc gctatcgcat ggtgaagggt 6840

cagcgtaaag agcttcctga aaggtggttc ttctactatt taggtactgg tcctcacgcc 6900

gatgctaaat ttaaagatag aatagatgga gttgtctggg ttgccaagga tggtgccatg 6960

aataagccaa ccacacttgg taatcgtggt gctaataatg aatccaaagc tttgaaattc 7020

gatggtaaag taccagtaga atttcaactt gaagtgaacc aatcaaggga caattcaagg 7080

tcacgctctc aatctagatc ccagtctaga aatagatctc aatctagagg aaggcaacaa 7140

tccaataaca agaaggatga cagtgtagaa caagctgttc ttgctgcact caaaaagtta 7200

ggtgttgaca cagaaaaaca acaacaacgc tctcgttcta aatccaagga acgtagcaac 7260

tctaagacaa gagacactac acctaagaat gaaaacaaac acacctggaa gagaactgca 7320

ggtaaaggtg atgtgacaaa attttatgga gctagaagta gttcagccaa ttttggtgac 7380

agcgatctcg ttgccaatgg tagcggtgcc aagcattacc cacaactggc tgaatgtgtt 7440

ccatctgtat ctagcattct gtttggaagc tattggactg caaaggaaga tggcgaccag 7500

attgaagtca cattcacaca taaataccac ttgccaaaag atgatcctaa gactggacaa 7560

ttccttcagc agattaatgc ctatgctcgt ccatcagagg tggcaaaaga acagagacaa 7620

cgcaaagctc gttctaaatc tgcagaaagg tcagagcaag aggttgtacc tgatgcatta 7680

acagaaaatt acacagatgt gtttgatgac acacaggttg agattattga tgaggtaacg 7740

aactaaacga atgctcgttt tcctccatgc tgtatttatc acagttttaa tcttactact 7800

aattggtaga ctccaattat tagaaagatt attacttaat cattccctca atcttaaaac 7860

tgtcaataat gttttaggtg tgactgacac tggtctgaaa gtaaattgct tacagctttt 7920

gaaaccagac tgtcttgatt ttaacatctt acataggagt ttggcagaaa ccagattact 7980

aaaagtagta cttcgagtaa tctttctagt attactaggg ttttgctgct acagattgtt 8040

agtcacatta atttagcatc atgaagtttt tgatttttgt actgtgtctt tctcttgtga 8100

atggatatgg aattagaaga agcatacaag aacatgaccc aaaagagtcc catgaacacc 8160

cgaccatgac atgggaatta ttggaaagat ttgttggaag taccttgtac atcacaacaa 8220

accaaatcct atctctacca actggagcgc aaatttattg tgatgagatt gaaggattcc 8280

aatgctcttg gcctggttat aaagcttatg ctcatgatca cattgattat cattttgatc 8340

tttccaaccc gttctattct tttgtaaata cattctacat ttctttaggt gatagaaaag 8400

aaaaaattta tcttagagtg gttggtgcaa caccaaaaga gaaaagattg aatgttggtt 8460

gtagaacatc tttctcagtt aacatgccaa ttggaactca gatttaccat gacaaagata 8520

tgcaatatct tgtcgaggga agacatcttg agtgtgctca cagagtttac tttgtgaagt 8580

actgtccaaa ccatgcacat ggttactgct ttaatgacag gctaaaggtt tataatcttg 8640

gtcgtgtcaa aagcagaaag gcttttgaga aaatcaacca acatcagaaa agtgagttgt 8700

aaggcaaccc gatgtttaaa actggttttt ccgaggaatt actgg 8745

<210> 2
<211> 4365
<212> DNA
<213> Unknown

<220>
<223> canine coronavirus

<400> 2
atgattgtga tcttaacttg cgtcttattg ttgtgctcgt accatactgt tgcgagtacg 60

acaaataatg attgtagaca agttaacgta acacaattag atggcaacga aaatctcatt 120

agagactttt tgtttcaaaa ctttaaagaa gaaggaactg tagttgttgg tggctattat 180

cctacagagg tgtggtacaa ctgttctaga acagcaccta ccactgccta tgagtatttt 240

aataacatac atgcatttta ttttgatatg gaagccatgg aaaatagcac tggtaatgca 300

cgtggtaaac cgttattgtt tcatgttcat ggtgagcctg ttagtgctat catatatata 360

tcggcttata gaaatgatgt acaacacagg ccacttttaa aacatgggtt agtgtgcata 420

actaaaactc gcaatattga ctataacagc ttcactagca gacagtggaa ttccatatgt 480

acaggtaatg acagaaaaat tcctttctct gtcataccca cggacaatgg aacaaaaatc 540

tatggacttg agtggaatga cgaatttgtt acagcctata ttagtggtca ttcttataat 600

tggaacatca ataataattg gtttaacaat gttacactac tttattcccg ctcaagtact 660

gctacatggc aacacagtgc tgcatatgtt taccaaggtg tttctaactt cacttattac 720

aagttaaata acaccaatgg tttaaaaact tatgaatttt gtgatgatta tgaatattgc 780

actggctacg ctactaatgt ctttgctccc actgtaggag gttacatacc tgatggattt 840

agttttaaca attggttttt gcttacaaat ggctccactt ttgttagtgg cagatttgta 900

acaaatcaac cattattagt taattgttta tggccagtgc ccagttttgg tgtggcagca 960

caagaatttt gttttgaagg tgcgcagttt agtcagtgca atggtgtgtc tttaaataac 1020

acagtggatg ttattagatt caatcttaat tttaccgcag atgtacaatc tggcatgggt 1080

gctacagtgt tctcattgaa tacaacaggt ggtgtcattc ttgaaatttc atgttataat 1140

gacacagtga gtgagtctag tttttacagt tatggtgaaa ttccatttgg cataactgat 1200

ggaccacggt actgttatgc actttacaat ggcacagctc ttaagtattt aggaacatta 1260

ccacctagtg taaaggaaat tgctattagt aagtggggcc atttttatat taatggttac 1320

aatttcttta gcacatttcc tattgattgt atatctttta atttaaccac tggtactagt 1380

ggagcttttt ggactattgc ttacacatcg tacactgaag cattagtaca agttgaaaac 1440

acagctatta aaaaggtgac gtattgtaac agtcacatta ataacattaa atgttctcaa 1500

cttactgcta atttgcaaaa tggtttttat cctgttgctt caagtgaagt tggtcttgtc 1560

aataagagtg ttgtgttact acctagtttc tattcacata ccagtgttaa tataactatt 1620

gatcttggta tgaagcgtag tggttatggt caacctatag cctcagcatt aagtaacatc 1680

acactaccga tgcaggataa taacaccgat gtgtattgca ttcgttctaa tcaattttca 1740

gtttacgtgc attccacttg taaaagttct ttatggacaa atgtgtttaa ttctgactgc 1800

acagattttt tacatgctac agctgttata aaaactggta cttgtccttt ctcatttgat 1860

aaattgaaca attacctaac ttttaacaag ttctgttttt cattgaatcc tgttggtgcc 1920

aattgcaaat ttgatgttgc tgcccgtaca agaaccaatg agcaggttgt tagaagttta 1980

tatgtgatat atgaagaagg agacaatata gtgggtgtac cgtctgataa tagtggtctg 2040

cacgatttgt cagtgttaca cttagactcc tgtacagatt acaatatata tggtagaact 2100

ggtgttggta ttattagaca aactaacagc acactactta gtggcttata ttacacatca 2160

ctatcaggtg atttgttagg ttttaaaaat gtcactgacg gtgttgtcta ttctgtaaca 2220

ccatgtgatg taagtgcaca agctgctgtt attgatggtg ccatagttgg agctatgact 2280

tccattaata gtgaactgtt aggtctaact cattggacaa caacacctaa tttttattat 2340

tactccatat ataattatac aaatgcgaga actcgtggca ctgcaatcga cagtaacgat 2400

gttgattgtg aacctatcat aacctattct aatataggtg tttgtaaaaa tggagctttg 2460

gtttttatta acgtcacaca ttctgatggt gacgttcaac caattagcac cggtaatgtc 2520

acgataccta caaattttac tatatctgtg caagtcgaat atattcaggt ttacactaca 2580

ccagtgtcaa tagactgttc aagatacgtc tgcaatggta atcctagatg caataaattg 2640

ttaacacaat atgtctctgc atgtcaaact attgagcaag cacttgcaat gggtgccaga 2700

cttgaaaaca tggaagttga ttccatgttg tttgtttctg aaaatgccct taaattggca 2760

tctgttgaag cattcaatag tacggaaact ctagatccta tttacaaaga atggcctaac 2820

attggtggtt cttggctagg aggtttaaaa gacatattgc catctcacaa tagcaaacgt 2880

aagtaccggt ctgctataga agatttgctt tttgataagg ttgtgacatc tggcttaggt 2940

acagtcgatg aagattacaa acgttgtacg ggtggctatg acatagctga cttagtgtgt 3000

gcacaatatt acaatggcat catggtgtta cctggtgtgg ctaatgatga caagatggct 3060

atgtacactg catctcttgc aggtggtata acattaggtg cacttggtgg tggtgcagtg 3120

tctatacctt ttgcagtagc agttcaggct agacttaatt atgtagcttt acaaactgac 3180

gtattgaaca aaaaccagca gattctggct aatgctttca atcaagcgat tggtaacatt 3240

acacaggcat ttggtaaggt taatgatgct atacatcaaa cgtcaaaagg tcttgctact 3300

gttgctaaag cattggcaaa agtgcaagat gttgttaaca cacaagggca agctttaagc 3360

cacctaacag tacaattgca aaataatttt caagccatta gtagttccat tagtgacatt 3420

tataacaggc ttgatgagtt gagtgcggat gcacaagttg acaggctgat tacaggacga 3480

cttacagcac ttaatgcatt tgtgtctcag actttaacca gacaagcaga ggttagggct 3540

agtagacaac ttgctaaaga caaagttaat gaatgcgtta ggtctcaatc ccaaagattt 3600

ggattctgtg gtaatggtac acatttgttt tcacttgcaa atgcagcacc aaatggcatg 3660

attttctttc acacagtgct attaccaaca gcttatgaaa ctgtgacggc ctggtcaggt 3720

atttgtgcat cagatggcga tcgcactttt ggacttgttg ttaaagatgt ccagctgacg 3780

ctatttcgca atttagatga caaattctat ttgacaccta gaactatgta tcagcctaga 3840

gttgcaacta gttctgattt tgttcaaatt gaagggtgtg atgtcttgtt tgtcaatgca 3900

actgtaattg agttgcctag tattatacct gattatatcg atattaatca gactgttcaa 3960

gacatattag aaaattacag accaaattgg acagtacctg aattaacact tgacattttc 4020

aacgcaacct acttaaacct gactggtgaa attaatgact tagaattcag gtcagaaaag 4080

ctacataaca ccacggtaga acttgctgtt ctcattgaca atattaacaa tacattagtc 4140

aatcttgaat ggctcaatag aattgaaact tatgtaaaat ggccttggta tgtatggcta 4200

ctaataggct tagtagtaat attttgcata ccattactgc tattttgctg ttgtagtaca 4260

ggttgctgtg ggtgcatagg ttgcttagga agttgttgtc actctatttg tagtagaaga 4320

caatttgaaa attatgaacc aattgagaaa gtgcatgtcc attaa 4365

<210> 3
<211> 216
<212> DNA
<213> Unknown

<220>
<223> canine coronavirus

<400> 3
atggacattg tcaaatctat tgacacatcc gtagacgctg tacttgacga acttgatcgt 60

gcatactttg ctgtaactct taaagtagag tttaagactg gtaaactact tgtgtgtata 120

ggttttggtg atacacctct tgaggctaag gataaagcat atgctaaact tggtttctct 180

attattgaag aagtcaatag tcatacagtt gtttga 216

<210> 4
<211> 69
<212> DNA
<213> Unknown

<220>
<223> canine coronavirus

<400> 4
atgctaaact tggtttctct attattgaag aagtcaatag tcatacagtt gtttgatatt 60

accttttga 69


<210> 5
<211> 735
<212> DNA
<213> Unknown

<220>
<223> canine coronavirus

<400> 5
atgattggtg gactttttct taacactctg agttttgtaa ttgttagcaa ccatgtcatt 60

gttaataata cagcaaatgt gcattctata caacaagaac atgttatagt acaacagcat 120

caggttgtta gtgctagaac acaaaattac tacccagagt tcagcatcgc tgtactcttt 180

gtattatttc tagctttgta ccgtagtaca aactttaaga cgtgtgtcga tatcttaatg 240

tttaagattg tatcaatgac acttataggg cctatgctta tagcatatgg ttactacatt 300

gatggcattg ttacaacaac tgtcttagct ttaagatttg tctacttatc atacttttgg 360

tatgttaata gtaggtttga attcatctta tacaatacaa cgacactcat gtttgtacat 420

ggcagagctg caccgtttat gagaagttct cacagctcta tttatgtcac attgtacggt 480

ggcataaatt atatgtttgt gaatgacctc acgttgcatt ttgtagaccc tatgcttgta 540

agcatagcaa tacgtggctt agctcatgct gatctaactg ttgttagagc agttgaactt 600

ctcaatggtg attttatcta tgtattttca caggagcccg tagtcggtgt ttacaatgca 660

gccttttctc aggcggttct aaacgaaatt gacttaaaag aagaagaaga agaccatacc 720

tatgacgttc cctag 735


<210> 6
<211> 306
<212> DNA
<213> Unknown

<220>
<223> canine coronavirus

<400> 6
atgctcgttt tcctccatgc tgtatttatc acagttttaa tcttactact aattggtaga 60

ctccaattat tagaaagatt attacttaat cattccctca atcttaaaac tgtcaataat 120

gttttaggtg tgactgacac tggtctgaaa gtaaattgct tacagctttt gaaaccagac 180

tgtcttgatt ttaacatctt acataggagt ttggcagaaa ccagattact aaaagtagta 240

cttcgagtaa tctttctagt attactaggg ttttgctgct acagattgtt agtcacatta 300

atttag 306

<210>  7
<211>  642
<212>  DNA
<213>  Unknown

<220>
<223>  canine coronavirus

<400>  7
atgaagtttt tgatttttgt actgtgtctt tctcttgtga atggatatgg aattagaaga      60

agcatacaag aacatgaccc aaaagagtcc catgaacacc cgaccatgac atgggaatta     120

ttggaaagat ttgttggaag taccttgtac atcacaacaa accaaatcct atctctacca     180

actggagcgc aaatttattg tgatgagatt gaaggattcc aatgctcttg gcctggttat     240

aaagcttatg ctcatgatca cattgattat cattttgatc tttccaaccc gttctattct     300

tttgtaaata cattctacat ttctttaggt gatagaaaag aaaaaattta tcttagagtg     360

gttggtgcaa caccaaaaga gaaaagattg aatgttggtt gtagaacatc tttctcagtt     420

aacatgccaa ttggaactca gatttaccat gacaaagata tgcaatatct tgtcgaggga     480

agacatcttg agtgtgctca cagagtttac tttgtgaagt actgtccaaa ccatgcacat     540

ggttactgct ttaatgacag gctaaaggtt tataatcttg gtcgtgtcaa aagcagaaag     600

gcttttgaga aaatcaacca acatcagaaa agtgagttgt aa                        642


<210>  8
<211>  249
<212>  DNA
<213>  Unknown

<220>
<223>  canine coronavirus

<400>  8
atgacgttcc ctagggcatt gactgtcata gatgacaatg gaatggtcat tagtatcatt      60

ttctggttcc tgttgataat tatattgata ttactttcaa tagcattgct aaatataatt     120

aagctatgca tggtatgttg taacttagga agaacagtta ttattgttcc agcgcaacat     180

gcctatgatg cctataagaa ttttatgcga attaaagcat ataaccccga cgaagcactc     240

cttgtttga                                                             249


<210>  9
<211>  789
<212>  DNA
<213>  Unknown

<220>
<223>  canine coronavirus

<400>  9
atgaagattt tgttagtatt agcgtgtgca attgcatgcg catgtggtga acgttattgc      60

gctatgaaat ctgactcaga tacttcgtgt cgcaatggta ccactactga ttgcgaatca 120

tgcttcaacg gaggtgatct tatttggtat cttgcaaact ggaacttcag ctggtctgta 180

atattgatcg tttttataac ggttttacaa tatggtagac ctcaatttag ctggttcgtg 240

tatggcatta aaatgcttat tatgtggcta ctatggccca ttgttctggc tcttacgatt 300

tttaatgcat actcggaata cgaagtttcc agatatgtaa tgttcggctt tagtgttgca 360

ggtgcaattg ttacatttat actttggatt atgtattttg ttagatccat tcagttatac 420

agaaggacta agtcttggtg gtctttcaac cctgaaacta acgcaattct ttgcgttagt 480

gcattaggaa ggagctatgt gcttcctctt gaaggtgtgc caactggtgt cactttaaca 540

ttgctctcag ggaatttgta cgctgaaggg ttcaaaattg caggtggtat gaacatcgac 600

aatttgccaa agtacgtaat ggttgcatta cctagcagga ccattgtcta cacacttgtt 660

ggtaagaaat tgaaagcaag tagtgcaaca ggatgggctt actatgtaaa atctaaagct 720

ggtgattact caacagatgc acgaactgac aatttgagtg agcaagaaaa attattacat 780

atggtataa 789

<210> 10
<211> 1149
<212> DNA
<213> Unknown

<220>
<223> canine coronavirus

<400> 10
atggccaacc agggacaacg cgttagctgg ggagatgaat ctaccaaaaa gcgtggtcgt 60

tccaattctc gtggccggaa gaataatact atacctcttt cattcttcaa ccccattacc 120

ctccaacaag gttcaaaatt ttggaactta tgtccgagag actttgtacc caaaggaata 180

ggtaacaagg atcaacagat tggttattgg aatagacaaa ctcgctatcg catggtgaag 240

ggtcagcgta aagagcttcc tgaaaggtgg ttcttctact atttaggtac tggtcctcac 300

gccgatgcta aatttaaaga tagaatagat ggagttgtct gggttgccaa ggatggtgcc 360

atgaataagc caaccacact tggtaatcgt ggtgctaata atgaatccaa agctttgaaa 420

ttcgatggta aagtaccagt agaatttcaa cttgaagtga accaatcaag ggacaattca 480

aggtcacgct ctcaatctag atcccagtct agaaatagat ctcaatctag aggaaggcaa 540

caatccaata acaagaagga tgacagtgta gaacaagctg ttcttgctgc actcaaaaag 600

ttaggtgttg acacagaaaa acaacaacaa cgctctcgtt ctaaatccaa ggaacgtagc 660

aactctaaga caagagacac tacacctaag aatgaaaaca aacacacctg gaagagaact 720

gcaggtaaag gtgatgtgac aaaattttat ggagctagaa gtagttcagc caattttggt 780

gacagcgatc tcgttgccaa tggtagcggt gccaagcatt acccacaact ggctgaatgt 840

gttccatctg tatctagcat tctgtttgga agctattgga ctgcaaagga agatggcgac 900

cagattgaag tcacattcac acataaatac cacttgccaa aagatgatcc taagactgga 960

caattccttc agcagattaa tgcctatgct cgtccatcag aggtggcaaa agaacagaga 1020

caacgcaaag ctcgttctaa atctgcagaa aggtcagagc aagaggttgt acctgatgca 1080

ttaacagaaa attacacaga tgtgtttgat gacacacagg ttgagattat tgatgaggta 1140

acgaactaa 1149

<210> 11
<211> 1454
<212> PRT
<213> Unknown

<220>
<223> canine coronavirus

<400> 11

Met Ile Val Ile Leu Thr Cys Val Leu Leu Leu Cys Ser Tyr His Thr
1               5                   10                  15

Val Ala Ser Thr Thr Asn Asn Asp Cys Arg Gln Val Asn Val Thr Gln
            20                  25                  30

Leu Asp Gly Asn Glu Asn Leu Ile Arg Asp Phe Leu Phe Gln Asn Phe
        35                  40                  45

Lys Glu Glu Gly Thr Val Val Val Gly Gly Tyr Tyr Pro Thr Glu Val
    50                  55                  60

Trp Tyr Asn Cys Ser Arg Thr Ala Pro Thr Thr Ala Tyr Glu Tyr Phe
65                  70                  75                  80

Asn Asn Ile His Ala Phe Tyr Phe Asp Met Glu Ala Met Glu Asn Ser
                85                  90                  95

Thr Gly Asn Ala Arg Gly Lys Pro Leu Leu Phe His Val His Gly Glu
            100                 105                 110

Pro Val Ser Ala Ile Ile Tyr Ile Ser Ala Tyr Arg Asn Asp Val Gln
        115                 120                 125

His Arg Pro Leu Leu Lys His Gly Leu Val Cys Ile Thr Lys Thr Arg
    130                 135                 140

Asn Ile Asp Tyr Asn Ser Phe Thr Ser Arg Gln Trp Asn Ser Ile Cys
145                 150                 155                 160

Thr Gly Asn Asp Arg Lys Ile Pro Phe Ser Val Ile Pro Thr Asp Asn
                165                 170                 175

Gly Thr Lys Ile Tyr Gly Leu Glu Trp Asn Asp Glu Phe Val Thr Ala
            180                 185                 190

Tyr Ile Ser Gly His Ser Tyr Asn Trp Asn Ile Asn Asn Asn Trp Phe
        195                 200                 205

Asn Asn Val Thr Leu Leu Tyr Ser Arg Ser Ser Thr Ala Thr Trp Gln
    210                 215                 220

His Ser Ala Ala Tyr Val Tyr Gln Gly Val Ser Asn Phe Thr Tyr Tyr

225                 230                 235                 240

Lys Leu Asn Asn Thr Asn Gly Leu Lys Thr Tyr Glu Phe Cys Asp Asp
                245                 250                 255

Tyr Glu Tyr Cys Thr Gly Tyr Ala Thr Asn Val Phe Ala Pro Thr Val
            260                 265                 270

Gly Gly Tyr Ile Pro Asp Gly Phe Ser Phe Asn Asn Trp Phe Leu Leu
        275                 280                 285

Thr Asn Gly Ser Thr Phe Val Ser Gly Arg Phe Val Thr Asn Gln Pro
    290                 295                 300

Leu Leu Val Asn Cys Leu Trp Pro Val Pro Ser Phe Gly Val Ala Ala
305                 310                 315                 320

Gln Glu Phe Cys Phe Glu Gly Ala Gln Phe Ser Gln Cys Asn Gly Val
                325                 330                 335

Ser Leu Asn Asn Thr Val Asp Val Ile Arg Phe Asn Leu Asn Phe Thr
            340                 345                 350

Ala Asp Val Gln Ser Gly Met Gly Ala Thr Val Phe Ser Leu Asn Thr
        355                 360                 365

Thr Gly Gly Val Ile Leu Glu Ile Ser Cys Tyr Asn Asp Thr Val Ser
    370                 375                 380

Glu Ser Ser Phe Tyr Ser Tyr Gly Glu Ile Pro Phe Gly Ile Thr Asp
385                 390                 395                 400

Gly Pro Arg Tyr Cys Tyr Ala Leu Tyr Asn Gly Thr Ala Leu Lys Tyr
                405                 410                 415

Leu Gly Thr Leu Pro Pro Ser Val Lys Glu Ile Ala Ile Ser Lys Trp
            420                 425                 430

Gly His Phe Tyr Ile Asn Gly Tyr Asn Phe Phe Ser Thr Phe Pro Ile
        435                 440                 445

Asp Cys Ile Ser Phe Asn Leu Thr Thr Gly Thr Ser Gly Ala Phe Trp
    450                 455                 460

Thr Ile Ala Tyr Thr Ser Tyr Thr Glu Ala Leu Val Gln Val Glu Asn
465                 470                 475                 480

Thr Ala Ile Lys Lys Val Thr Tyr Cys Asn Ser His Ile Asn Asn Ile
                485                 490                 495

Lys Cys Ser Gln Leu Thr Ala Asn Leu Gln Asn Gly Phe Tyr Pro Val
            500                 505                 510

Ala Ser Ser Glu Val Gly Leu Val Asn Lys Ser Val Val Leu Leu Pro
        515                 520                 525

Ser Phe Tyr Ser His Thr Ser Val Asn Ile Thr Ile Asp Leu Gly Met
    530                 535                 540

Lys Arg Ser Gly Tyr Gly Gln Pro Ile Ala Ser Ala Leu Ser Asn Ile
545                 550                 555                 560

Thr Leu Pro Met Gln Asp Asn Asn Thr Asp Val Tyr Cys Ile Arg Ser
                565                 570                 575

Asn Gln Phe Ser Val Tyr Val His Ser Thr Cys Lys Ser Ser Leu Trp
            580                 585                 590

Asp Asn Val Phe Asn Ser Asp Cys Thr Asp Phe Leu His Ala Thr Ala
        595                 600                 605

Val Ile Lys Thr Gly Thr Cys Pro Phe Ser Phe Asp Lys Leu Asn Asn
    610                 615                 620

Tyr Leu Thr Phe Asn Lys Phe Cys Phe Ser Leu Asn Pro Val Gly Ala
625                 630                 635                 640

Asn Cys Lys Phe Asp Val Ala Ala Arg Thr Arg Thr Asn Glu Gln Val
                645                 650                 655

Val Arg Ser Leu Tyr Val Ile Tyr Glu Glu Gly Asp Asn Ile Val Gly
            660                 665                 670

Val Pro Ser Asp Asn Ser Gly Leu His Asp Leu Ser Val Leu His Leu
        675                 680                 685

Asp Ser Cys Thr Asp Tyr Asn Ile Tyr Gly Arg Thr Gly Val Gly Ile
    690                 695                 700

Ile Arg Gln Thr Asn Ser Thr Leu Leu Ser Gly Leu Tyr Tyr Thr Ser
705                 710                 715                 720

Leu Ser Gly Asp Leu Leu Gly Phe Lys Asn Val Thr Asp Gly Val Val
                725                 730                 735

Tyr Ser Val Thr Pro Cys Asp Val Ser Ala Gln Ala Ala Val Ile Asp
            740                 745                 750

Gly Ala Ile Val Gly Ala Met Thr Ser Ile Asn Ser Glu Leu Leu Gly
        755                 760                 765

Leu Thr His Trp Thr Thr Thr Pro Asn Phe Tyr Tyr Tyr Ser Ile Tyr
    770                 775                 780

Asn Tyr Thr Asn Ala Arg Thr Arg Gly Thr Ala Ile Asp Ser Asn Asp
785                 790                 795                 800

Val Asp Cys Glu Pro Ile Ile Thr Tyr Ser Asn Ile Gly Val Cys Lys
                805                 810                 815

Asn Gly Ala Leu Val Phe Ile Asn Val Thr His Ser Asp Gly Asp Val
            820                 825                 830

Gln Pro Ile Ser Thr Gly Asn Val Thr Ile Pro Thr Asn Phe Thr Ile
        835                 840                 845

Ser Val Gln Val Glu Tyr Ile Gln Val Tyr Thr Thr Pro Val Ser Ile
    850                 855                 860

Asp Cys Ser Arg Tyr Val Cys Asn Gly Asn Pro Arg Cys Asn Lys Leu
865                 870                 875                 880

Leu Thr Gln Tyr Val Ser Ala Cys Gln Thr Ile Glu Gln Ala Leu Ala
                885                 890                 895

Met Gly Ala Arg Leu Glu Asn Met Glu Val Asp Ser Met Leu Phe Val
            900                 905                 910

Ser Glu Asn Ala Leu Lys Leu Ala Ser Val Glu Ala Phe Asn Ser Thr
        915                 920                 925

Glu Thr Leu Asp Pro Ile Tyr Lys Glu Trp Pro Asn Ile Gly Gly Ser
    930                 935                 940

Trp Leu Gly Gly Leu Lys Asp Ile Leu Pro Ser His Asn Ser Lys Arg
945                 950                 955                 960

Lys Tyr Arg Ser Ala Ile Glu Asp Leu Leu Phe Asp Lys Val Val Thr
                965                 970                 975

Ser Gly Leu Gly Thr Val Asp Glu Asp Tyr Lys Arg Cys Thr Gly Gly
            980                 985                 990

Tyr Asp Ile Ala Asp Leu Val Cys  Ala Gln Tyr Tyr Asn  Gly Ile Met
        995                 1000                 1005

Val Leu  Pro Gly Val Ala Asn  Asp Asp Lys Met Ala  Met Tyr Thr
    1010                 1015                 1020

Ala Ser  Leu Ala Gly Gly Ile  Thr Leu Gly Ala Leu  Gly Gly Gly
    1025                 1030                 1035

Ala Val  Ser Ile Pro Phe Ala  Val Ala Val Gln Ala  Arg Leu Asn
    1040                 1045                 1050

Tyr Val  Ala Leu Gln Thr Asp  Val Leu Asn Lys Asn  Gln Gln Ile
    1055                 1060                 1065

Leu Ala  Asn Ala Phe Asn Gln  Ala Ile Gly Asn Ile  Thr Gln Ala
    1070                 1075                 1080

Phe Gly  Lys Val Asn Asp Ala  Ile His Gln Thr Ser  Lys Gly Leu
    1085                 1090                 1095

Ala Thr  Val Ala Lys Ala Leu  Ala Lys Val Gln Asp  Val Val Asn
    1100                 1105                 1110

Thr Gln  Gly Gln Ala Leu Ser  His Leu Thr Val Gln  Leu Gln Asn
    1115                 1120                 1125

Asn Phe  Gln Ala Ile Ser Ser  Ser Ile Ser Asp Ile  Tyr Asn Arg
    1130                 1135                 1140

Leu Asp  Glu Leu Ser Ala Asp  Ala Gln Val Asp Arg  Leu Ile Thr
    1145                 1150                 1155

Gly Arg Leu Thr Ala Leu Asn Ala Phe Val Ser Gln Thr Leu Thr
    1160                1165                1170

Arg Gln Ala Glu Val Arg Ala Ser Arg Gln Leu Ala Lys Asp Lys
    1175                1180                1185

Val Asn Glu Cys Val Arg Ser Gln Ser Gln Arg Phe Gly Phe Cys
    1190                1195                1200

Gly Asn Gly Thr His Leu Phe Ser Leu Ala Asn Ala Ala Pro Asn
    1205                1210                1215

Gly Met Ile Phe Phe His Thr Val Leu Leu Pro Thr Ala Tyr Glu
    1220                1225                1230

Thr Val Thr Ala Trp Ser Gly Ile Cys Ala Ser Asp Gly Asp Arg
    1235                1240                1245

Thr Phe Gly Leu Val Val Lys Asp Val Gln Leu Thr Leu Phe Arg
    1250                1255                1260

Asn Leu Asp Asp Lys Phe Tyr Leu Thr Pro Arg Thr Met Tyr Gln
    1265                1270                1275

Pro Arg Val Ala Thr Ser Ser Asp Phe Val Gln Ile Glu Gly Cys
    1280                1285                1290

Asp Val Leu Phe Val Asn Ala Thr Val Ile Glu Leu Pro Ser Ile
    1295                1300                1305

Ile Pro Asp Tyr Ile Asp Ile Asn Gln Thr Val Gln Asp Ile Leu
    1310                1315                1320

Glu Asn Tyr Arg Pro Asn Trp Thr Val Pro Glu Leu Thr Leu Asp
    1325                1330                1335

Ile Phe Asn Ala Thr Tyr Leu Asn Leu Thr Gly Glu Ile Asn Asp
    1340                1345                1350

Leu Glu Phe Arg Ser Glu Lys Leu His Asn Thr Thr Val Glu Leu
    1355                1360                1365

Ala Val Leu Ile Asp Asn Ile Asn Asn Thr Leu Val Asn Leu Glu
    1370                1375                1380

Trp Leu Asn Arg Ile Glu Thr Tyr Val Lys Trp Pro Trp Tyr Val
    1385                1390                1395

Trp Leu  Leu Ile Gly Leu Val  Val Ile Phe Cys Ile  Pro Leu Leu
    1400                 1405                 1410

Leu Phe  Cys Cys Cys Ser Thr  Gly Cys Cys Gly Cys  Ile Gly Cys
    1415                 1420                 1425

Leu Gly  Ser Cys Cys His Ser  Ile Cys Ser Arg Arg  Gln Phe Glu
    1430                 1435                 1440

Asn Tyr  Glu Pro Ile Glu Lys  Val His Val His
    1445                 1450

<210>  12
<211>  71
<212>  PRT
<213>  Unknown

<220>
<223>  canine coronavirus

<400>  12

Met Asp Ile Val Lys Ser Ile Asp Thr Ser Val Asp Ala Val Leu Asp
1               5                   10                  15

Glu Leu Asp Arg Ala Tyr Phe Ala Val Thr Leu Lys Val Glu Phe Lys
            20                  25                  30

Thr Gly Lys Leu Leu Val Cys Ile Gly Phe Gly Asp Thr Pro Leu Glu
        35                  40                  45

Ala Lys Asp Lys Ala Tyr Ala Lys Leu Gly Phe Ser Ile Ile Glu Glu
    50                  55                  60

Val Asn Ser His Thr Val Val
65                  70

<210>  13
<211>  22
<212>  PRT
<213>  Unknown

<220>
<223>  canine coronavirus

<400>  13

Met Leu Asn Leu Val Ser Leu Leu Leu Lys Lys Ser Ile Val Ile Gln
1               5                   10                  15

Leu Phe Asp Ile Thr Phe
            20

<210>  14
<211>  244
<212>  PRT
<213>  Unknown

<220>
<223>  canine coronavirus

<400>  14

Met Ile Gly Gly Leu Phe Leu Asn Thr Leu Ser Phe Val Ile Val Ser
1               5                   10                  15

Asn His Val Ile Val Asn Asn Thr Ala Asn Val His Ser Ile Gln Gln
            20                  25                  30

Glu His Val Ile Val Gln Gln His Gln Val Val Ser Ala Arg Thr Gln
        35                  40                  45

Asn Tyr Tyr Pro Glu Phe Ser Ile Ala Val Leu Phe Val Leu Phe Leu
    50                  55                  60

Ala Leu Tyr Arg Ser Thr Asn Phe Lys Thr Cys Val Asp Ile Leu Met
65                  70                  75                  80

Phe Lys Ile Val Ser Met Thr Leu Ile Gly Pro Met Leu Ile Ala Tyr
                85                  90                  95

Gly Tyr Tyr Ile Asp Gly Ile Val Thr Thr Thr Val Leu Ala Leu Arg
            100                 105                 110

Phe Val Tyr Leu Ser Tyr Phe Trp Tyr Val Asn Ser Arg Phe Glu Phe
        115                 120                 125

Ile Leu Tyr Asn Thr Thr Thr Leu Met Phe Val His Gly Arg Ala Ala
    130                 135                 140

Pro Phe Met Arg Ser Ser His Ser Ser Ile Tyr Val Thr Leu Tyr Gly
145                 150                 155                 160

Gly Ile Asn Tyr Met Phe Val Asn Asp Leu Thr Leu His Phe Val Asp
                165                 170                 175

Pro Met Leu Val Ser Ile Ala Ile Arg Gly Leu Ala His Ala Asp Leu
            180                 185                 190

Thr Val Val Arg Ala Val Glu Leu Leu Asn Gly Asp Phe Ile Tyr Val
        195                 200                 205

Phe Ser Gln Glu Pro Val Val Gly Val Tyr Asn Ala Ala Phe Ser Gln
    210                 215                 220

Ala Val Leu Asn Glu Ile Asp Leu Lys Glu Glu Glu Glu Asp His Thr
225                 230                 235                 240

Tyr Asp Val Pro

<210>  15
<211>  101
<212>  PRT
<213>  Unknown

<220>
<223>  canine coronavirus

<400>  15

Met Leu Val Phe Leu His Ala Val Phe Ile Thr Val Leu Ile Leu Leu
1               5                   10                  15

Leu Ile Gly Arg Leu Gln Leu Leu Glu Arg Leu Leu Leu Asn His Ser
            20                  25                  30

Leu Asn Leu Lys Thr Val Asn Asn Val Leu Gly Val Thr Asp Thr Gly
        35                  40                  45

Leu Lys Val Asn Cys Leu Gln Leu Leu Lys Pro Asp Cys Leu Asp Phe
    50                  55                  60

Asn Ile Leu His Arg Ser Leu Ala Glu Thr Arg Leu Leu Lys Val Val
65                  70                  75                  80

Leu Arg Val Ile Phe Leu Val Leu Leu Gly Phe Cys Cys Tyr Arg Leu
                85                  90                  95

Leu Val Thr Leu Ile
            100

<210>  16
<211>  213
<212>  PRT
<213>  Unknown

<220>
<223>  canine coronavirus

<400>  16

Met Lys Phe Leu Ile Phe Val Leu Cys Leu Ser Leu Val Asn Gly Tyr
1               5                   10                  15

Gly Ile Arg Arg Ser Ile Gln Glu His Asp Pro Lys Glu Ser His Glu
            20                  25                  30

His Pro Thr Met Thr Trp Glu Leu Leu Glu Arg Phe Val Gly Ser Thr
        35                  40                  45

Leu Tyr Ile Thr Thr Asn Gln Ile Leu Ser Leu Pro Thr Gly Ala Gln
    50                  55                  60

Ile Tyr Cys Asp Glu Ile Glu Gly Phe Gln Cys Ser Trp Pro Gly Tyr
65                  70                  75                  80

Lys Ala Tyr Ala His Asp His Ile Asp Tyr His Phe Asp Leu Ser Asn
                85                  90                  95

Pro Phe Tyr Ser Phe Val Asn Thr Phe Tyr Ile Ser Leu Gly Asp Arg
            100                 105                 110

Lys Glu Lys Ile Tyr Leu Arg Val Val Gly Ala Thr Pro Lys Glu Lys
        115                 120                 125

Arg Leu Asn Val Gly Cys Arg Thr Ser Phe Ser Val Asn Met Pro Ile
    130                 135                 140

Gly Thr Gln Ile Tyr His Asp Lys Asp Met Gln Tyr Leu Val Glu Gly
145                 150                 155                 160

Arg His Leu Glu Cys Ala His Arg Val Tyr Phe Val Lys Tyr Cys Pro
                165                 170                 175

Asn His Ala His Gly Tyr Cys Phe Asn Asp Arg Leu Lys Val Tyr Asn
            180                 185                 190

Leu Gly Arg Val Lys Ser Arg Lys Ala Phe Glu Lys Ile Asn Gln His
        195                 200                 205

Gln Lys Ser Glu Leu
    210

<210>  17
<211>  82
<212>  PRT
<213>  Unknown

<220>
<223>  canine coronavirus

<400>  17

Met Thr Phe Pro Arg Ala Leu Thr Val Ile Asp Asp Asn Gly Met Val
1               5                   10                  15

Ile Ser Ile Ile Phe Trp Phe Leu Leu Ile Ile Ile Leu Ile Leu Leu
            20                  25                  30

Ser Ile Ala Leu Leu Asn Ile Ile Lys Leu Cys Met Val Cys Cys Asn
        35                  40                  45

Leu Gly Arg Thr Val Ile Ile Val Pro Ala Gln His Ala Tyr Asp Ala
    50                  55                  60

Tyr Lys Asn Phe Met Arg Ile Lys Ala Tyr Asn Pro Asp Glu Ala Leu
65                  70                  75                  80

Leu Val

<210>  18
<211>  382
<212>  PRT
<213>  Unknown

<220>
<223>  canine coronavirus

<400>  18

Met Ala Asn Gln Gly Gln Arg Val Ser Trp Gly Asp Glu Ser Thr Lys
1               5                   10                  15

Lys Arg Gly Arg Ser Asn Ser Arg Gly Arg Lys Asn Asn Thr Ile Pro
            20                  25                  30

Leu Ser Phe Phe Asn Pro Ile Thr Leu Gln Gln Gly Ser Lys Phe Trp
        35                  40                  45

Asn Leu Cys Pro Arg Asp Phe Val Pro Lys Gly Ile Gly Asn Lys Asp
    50                  55                  60

Gln Gln Ile Gly Tyr Trp Asn Arg Gln Thr Arg Tyr Arg Met Val Lys
65                  70                  75                  80

Gly Gln Arg Lys Glu Leu Pro Glu Arg Trp Phe Phe Tyr Tyr Leu Gly
                85                  90                  95

Thr Gly Pro His Ala Asp Ala Lys Phe Lys Asp Arg Ile Asp Gly Val
            100                 105                 110

Val Trp Val Ala Lys Asp Gly Ala Met Asn Lys Pro Thr Thr Leu Gly
        115                 120                 125

Asn Arg Gly Ala Asn Asn Glu Ser Lys Ala Leu Lys Phe Asp Gly Lys
    130                 135                 140

Val Pro Val Glu Phe Gln Leu Glu Val Asn Gln Ser Arg Asp Asn Ser
145                 150                 155                 160

Arg Ser Arg Ser Gln Ser Arg Ser Gln Ser Arg Asn Arg Ser Gln Ser
                165                 170                 175

Arg Gly Arg Gln Gln Ser Asn Asn Lys Lys Asp Asp Ser Val Glu Gln
            180                 185                 190

Ala Val Leu Ala Ala Leu Lys Lys Leu Gly Val Asp Thr Glu Lys Gln
        195                 200                 205

Gln Gln Arg Ser Arg Ser Lys Ser Lys Glu Arg Ser Asn Ser Lys Thr
    210                 215                 220

Arg Asp Thr Thr Pro Lys Asn Glu Asn Lys His Thr Trp Lys Arg Thr
225                 230                 235                 240

Ala Gly Lys Gly Asp Val Thr Lys Phe Tyr Gly Ala Arg Ser Ser Ser
                245                 250                 255

Ala Asn Phe Gly Asp Ser Asp Leu Val Ala Asn Gly Ser Gly Ala Lys
            260                 265                 270

His Tyr Pro Gln Leu Ala Glu Cys Val Pro Ser Val Ser Ser Ile Leu
        275                 280                 285

Phe Gly Ser Tyr Trp Thr Ala Lys Glu Asp Gly Asp Gln Ile Glu Val
    290                 295                 300

Thr Phe Thr His Lys Tyr His Leu Pro Lys Asp Asp Pro Lys Thr Gly
305                 310                 315                 320

Gln Phe Leu Gln Gln Ile Asn Ala Tyr Ala Arg Pro Ser Glu Val Ala
                325                 330                 335

Lys Glu Gln Arg Gln Arg Lys Ala Arg Ser Lys Ser Ala Glu Arg Ser
            340                 345                 350

Glu Gln Glu Val Val Pro Asp Ala Leu Thr Glu Asn Tyr Thr Asp Val
        355                 360                 365

Phe Asp Asp Thr Gln Val Glu Ile Ile Asp Glu Val Thr Asn
    370                 375                 380

<210>  19
<211>  262
<212>  PRT
<213>  Unknown

<220>
<223>  canine coronavirus

<400>  19

Met Lys Ile Leu Leu Val Leu Ala Cys Ala Ile Ala Cys Ala Cys Gly
1               5                   10                  15

Glu Arg Tyr Cys Ala Met Lys Ser Asp Ser Asp Thr Ser Cys Arg Asn
            20                  25                  30

Gly Thr Thr Thr Asp Cys Glu Ser Cys Phe Asn Gly Gly Asp Leu Ile
        35                  40                  45

Trp Tyr Leu Ala Asn Trp Asn Phe Ser Trp Ser Val Ile Leu Ile Val
    50                  55                  60

Phe Ile Thr Val Leu Gln Tyr Gly Arg Pro Gln Phe Ser Trp Phe Val
65                  70                  75                  80

Tyr Gly Ile Lys Met Leu Ile Met Trp Leu Leu Trp Pro Ile Val Leu
                85                  90                  95

Ala Leu Thr Ile Phe Asn Ala Tyr Ser Glu Tyr Glu Val Ser Arg Tyr
            100                 105                 110

Val Met Phe Gly Phe Ser Val Ala Gly Ala Ile Val Thr Phe Ile Leu
        115                 120                 125

Trp Ile Met Tyr Phe Val Arg Ser Ile Gln Leu Tyr Arg Arg Thr Lys
    130                 135                 140

Ser Trp Trp Ser Phe Asn Pro Glu Thr Asn Ala Ile Leu Cys Val Ser
145                 150                 155                 160

Ala Leu Gly Arg Ser Tyr Val Leu Pro Leu Glu Gly Val Pro Thr Gly
                165                 170                 175

Val Thr Leu Thr Leu Leu Ser Gly Asn Leu Tyr Ala Glu Gly Phe Lys
            180                 185                 190

Ile Ala Gly Gly Met Asn Ile Asp Asn Leu Pro Lys Tyr Val Met Val
        195                 200                 205

Ala Leu Pro Ser Arg Thr Ile Val Tyr Thr Leu Val Gly Lys Lys Leu
    210                 215                 220

Lys Ala Ser Ser Ala Thr Gly Trp Ala Tyr Tyr Val Lys Ser Lys Ala
225                 230                 235                 240

Gly Asp Tyr Ser Thr Asp Ala Arg Thr Asp Asn Leu Ser Glu Gln Glu
                245                 250                 255

Lys Leu Leu His Met Val
            260

## Description

The present invention relates generally to canine coronavirus (CCoV) infections, and specifically to a pantropic, highly pathogenic variant of CCoV type II that is responsible for acute, fatal disease in dogs. The invention also provides polynucleotides and proteins isolated from the canine coronavirus, and their use in the prophylaxis, therapy and diagnosis of canine infections.

### Background of the invention

Coronaviruses are large enveloped positive-stranded RNA viruses (1). Three different coronaviruses have been identified in dogs thus far (2,3). Canine coronavirus (CCoV) type I and type II are included in group 1 coronaviruses and their evolution is tightly intermingled with that of feline coronaviruses (FCoV type I and type II). FCoV type II has originated by heterologous recombination between CCoV type II and FCoV type I, while CCoV type I is genetically more similar to FCoV type I than to CCoV type II (3). In addition, two FCoV biotypes, that differ in their pathogenicity, have been observed in cats and the onset of acute fatal forms of diseases (referred to as feline infectious peritonitis) is accounted for by the onset of pantropic variants (able to disseminate throughout the organism) of enteric FCoVs, that are likely related to deletions/recombinations in the 3c and 7b genes at the 3' end of FCoV genome (4). Similarly, drastic shift of tissue tropisms in porcine and murine coronaviruses (5,6) and adaptation to humans of the recently-recognized SARS-associated coronavirus (SARS-CoV) (7) has been related to even minimal genome mutations and/or deletions. A third canine coronavirus, CRCoV, detected in the respiratory tract, shares up to 96.0% amino acid (aa) conservation in the spike protein S with bovine coronavirus, within group 2 coronaviruses, providing strong evidence for a recent host-species shift (2).

Coronavirus infection in dogs is usually restricted to the enteric tract. The infection is self-limiting and in general produces only mild or even asymptomatic forms of enteritis (8).

### Description of the invention

A highly pathogenic pantropic variant of canine coronavirus has been isolated from dogs presenting severe internal lesions. Experimental infection of dogs with the virus isolate resulted in a severe systemic disease that mimicked the clinical signs originally observed in the animals. The sequence of the 3' genome end of the pantropic CCoV strain was determined by RT-PCR amplification and sequencing of overlapping fragments. The structural and nonstructural proteins of the newly isolated CCoV strain displayed high sequence identity to those of other type II CCoVs, with the exceptions of nonstructural proteins (nsp) 3b and 3c, the sequences of which resulted significantly different in the isolated strain and in known type II canine coronaviruses, respectively.

In one aspect the present invention provides the partial genomic sequence of the newly isolated coronavirus strain (SEQ ID NO: 1), which can be used to prepare diagnostic reagents or tools for virus detection in dogs. Specifically, the nucleotide fragments of SEQ ID NO: 1, or sequences complementary thereto, can be used as diagnostic probes or PCR primers in a diagnostic method employing the polymerase chain reaction (PCR) technique to identify the presence of the virus in animal body fluids or tissues, e.g. for the screening of sera. In a typical application, tissue samples from a dog suspected to have come into contact with the coronavirus strain are subjected to PCR amplification using virus-specific primers. The latter are preferably selected from virus-specific genomic regions that allow to distinguish the coronavirus strain herein disclosed. The most distinctive and virus-specific genomic regions from which suitable PCR primers may be selected are those containing the sequences coding for the spike protein (S) (SEQ ID NO: 2), the non-structural proteins 3a, 3b, 3c, 7a and 7b (SEQ ID NO: 3, 4, 5, 6 and 7, respectively), the envelope protein E (SEQ ID NO: 8), the membrane protein M (SEQ ID NO: 9) and the nucleocapsid protein N (SEQ ID NO: 10). Particularly preferred are the genomic regions containing the sequence coding for the non-structural proteins 3b and 3c, which show significant alterations in the isolated coronavirus strain compared to phylogenetically-related viruses.

In a particularly preferred embodiment, the diagnostic primers or probes are designed to identify the 38-nucleotide deletion in the nsp3b gene.

Virus-specific proteins, preferably the non-structural proteins nsp3b and nsp3c, and antigenic peptides thereof, can be used to assemble immuno- or radioimmuno assays, such as ELISA or Western blot, or for the development of antibodies. The preferred antigens for use in immunoassays and for developing antibodies are selected from the structural and non-structural proteins herein provided, the sequences of which are listed in SEQ ID NO: 11 (spike protein S), SEQ ID NO: 12 (nsp3a), SEQ ID NO: 13 (nsp3b), SEQ ID NO: 14 (nsp3c), SEQ ID NO: 15 (nsp7a), SEQ ID NO: 16 (nsp7b), SEQ ID NO: 17 (envelope protein E), SEQ ID NO: 18 (nucleocapsid protein N), SEQ ID NO: 19 (membrane protein M). The virus-specific sequences of nsp3b and nsp3c (SEQ ID NOs: 13 and 14) identify preferred antigens for use in a diagnostic method according to the invention.

In a further aspect, the invention provides antibodies to one or more epitopes in the above identified amino acid sequences. The antibodies may be monoclonal, polyclonal or fragments such as Fab, Fv and scFv.

In a yet further embodiment, the invention provides a vaccine composition containing an immunogenic protein, which is preferably selected from SEQ ID NO: 11 through SEQ ID NO: 19, or an avirulent antigenic product obtained by attenuation or inactivation of the coronavirus pathogenic strain herein disclosed. The latter can be isolated from organs or tissues of infected animals, propagated in mammalian cells, recovered and concentrated to an appropriate viral titer, inactivated by chemical treatment or attenuated by repeated passages in suitable cells, especially in feline or canine cells. The coronavirus strain can be easily identified by detection of specific molecular markers, such as proteins nsp 3b and 3c (SEQ ID NOs: 13 and 14). The preparation of a vaccine composition containing, in addition to the active ingredient, appropriate vehicles, excipients or adjuvants, is within the skill of the art. The vaccine composition may be used for the prohylaxis or treatment of infections caused by the pantropic, highly-virulent CCoV strain in dogs, particularly in young animals and pups.

### Detailed description of the invention

Recently, a severe outbreak of a fatal systemic disease occurred in a pet shop in Bari, Italy. Clinical signs were observed initially in three Miniature Pinscher and in a Spaniel Cocker, 45 and 53 days of age, respectively, and consisted of fever (39.5-40°C), lethargy, inappetance, vomiting, hemorrhagic diarrhea, and neurological signs (ataxia, seizures) with death after 2 days. After few days, the same signs were observed in two additional 45-day-old Miniature Pinschers and in a 56-day-old Pekinese dog. At necropsy, the dogs showed hemorrhagic enteritis, abundant serosanguineous fluid in the abdominal cavity and severe lesions in the parenchymatous organs. The lungs had multiple, patchy, red areas of consolidation. The liver was yellowishbrown and congested, with hemorrhages on its surface, while the spleen was enlarged with subcapsular hemorrhages. Variable gross changes in other organs included multifocal hemorrhagic renal cortical infarcts and petechial hemorrhages on the lymph node surface.

Virological and bacteriological investigations on the parenchymatous organs failed to detect common canine pathogens, notably canine parvovirus type-2, canine distemper virus, canine adenovirus type-1 and type-2, whereas CCoV type I and type II were identified in the intestinal content of all pups by genotype-specific real-time RT-PCR assays (9). Unexpectedly, CCoV type II RNA was also detected in lungs (median titers of 1.08×10⁶ RNA copies/µl of template), spleen (median titers of 4.46×10⁶ RNA copies/µl of template), liver (median titers of 9.02×10⁴ RNA copies/µl of template), kidney (median titers of 7.54×10⁴ RNA copies/µl of template), and brain (median titers of 5.23×10³ RNA copies/µl of template). Virus-induced cytopthatic effect was observed in A-72 cells and CCoV type II strain (CB/05) was isolated from all the examined tissues but brain.

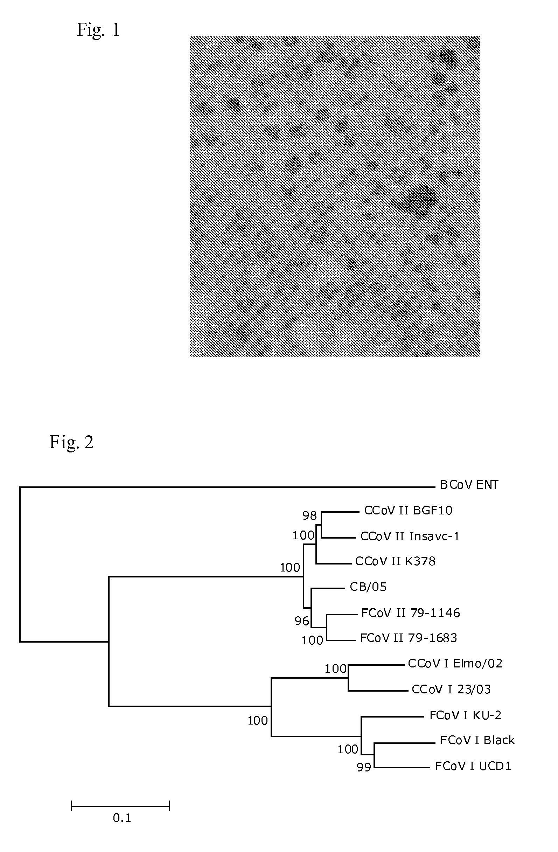

Immunohistochemistry using a CCoV-specific monoclonal antibody detected CCoV antigen in the organs with gross lesions that were examined (lungs, kidneys, liver, spleen, gut and lymph nodes (Fig. 1).

The sequence of the 3' genome end (8.8 kb) of the pantropic CCoV strain was determined by RT-PCR amplification and sequencing of overlapping fragments. The structural proteins S, E, M, N displayed a high degree of aa identity to the cognate ORFs of CCoV type II. The S protein of strain CB/05 displayed the highest identity to FCoV type II strain 79-1146 (Fig. 2). Due to a lack of data from the 3' end of the CCoV genome in the genes encoding for nonstructural proteins (3a, 3b, 3c, 7a and 7b), comparison of strain CB/05 was possible only with CCoV type II strains Insavc-1 (10) and BGF (11) and with CCoV type I strains Elmo/02 and 23/03 (3,12). The nonstructural proteins (nsp) 3a, 7a and 7b presented slight sequence-alterations. The nsp3b was 49-aa shorter (22 aa) than expected due to the presence of a 38-nt deletion and to a frame shift in the sequence downstream the deletion that introduced an early stop codon. The nsp3c (244 aa) was 6-aa shorter and 79-aa longer than the cognate proteins of the enteropathogen strain BGF and of the attenuated strain Insavc-1a, respectively.

To confirm the pathogenic potential of strain CB/05, two 6-month-old dogs were infected experimentally (authorization no. 67/2002-C released by Ministry of Health of Italy). Two ml of the cryolysate of a lung-derived 1^{st} virus passage in A-72 cells were administered intranasally to the dogs. The cell cryolysate tested negative to other common canine pathogens and displayed an infectivity dose of 10^{5.50} TCID₅₀/50 µl on A-72 cells, and a titer of 1.18×10⁷ RNA copies/µl of template by real-time RT-PCR. The virus was re-isolated from the experimentally-infected dogs. Severe clinical signs, characterized by pyrexia (39.8-40.1°C), anorexia, depression, vomiting, diarrhea and leukopenia, were observed, that persisted 8-10 days. Despite the severe symptoms, the dogs slowly recovered from illness.

### Description of the Figures

**Figure 1****.** Immunohistochemistry on a section of lung tissue. CCoV antigen detection (brown staining) by monoclonal antibody. 400X.

**Figure 2****.** Neighbor-joining tree of the S protein of canine and feline coronaviruses. For the phylogenetic analysis the following reference strains were used: CCoV type I strains Elmo/02 (accession no AY307020) and 23/03 (AY307021); CCoV type II strains Insavc-1 (D13096) and K378 (X77047); FCoV type I strains KU-2 (D32044), Black (AB088223) and UCD-1 (AB088222); FCoV type II strains 79-1146 (X06170) and 79-1683 (X80799); bovine coronavirus (BCoV) strain ENT (NC_003045). The tree is rooted on BCoV-ENT and drawn to scale. A statistical support was provided by bootstrapping over 100 replicates.

### References

1. Lai MMC, Holmes KV. Coronaviridae: The viruses and their replication. In: Knipe DM, Howley PM, editors. Fields Virology, 4th edition. Philadelphia, PA: Lippincott Williams and Wilkins. 2001; p.1163-85.
2. Erles K, Toomey C, Brooks HW, Brownlie. J. Detection of a group 2 coronavirus in dogs with canine infectious respiratory disease. Virology. 2003;310:216-23.
3. Pratelli A, Martella V, Decaro N, Tinelli A, Camero M, Cirone F, et al. Genetic diversity of a canine coronavirus detected in pups with diarrhoea in Italy. J Virol Methods. 2003;110:9-17.
4. Vennema H, Poland A, Foley J, Pedersen NC. Feline infectious peritonitis viruses arise by mutation from endemic feline enteric coronaviruses. Virology. 1998;243:150-7.
5. Laude H, Van Reeth K, Pensaert M. Porcine respiratory coronavirus: molecular features and virus-host interactions. Vet Res. 1993;24:125-50.
6. Haspel MV, Lampert PW, Oldstone MB. Temperature-sensitive mutants of mouse hepatitis virus produce a high incidence of demyelination. Proc Natl Acad Sci USA. 1978;75:4033-36.
7. Guan Y, Zheng BJ, He YQ, Liu XL, Zhuang ZX, Cheung CL, et al. Isolation and characterization of viruses related to the SARS coronavirus from animals in southern China. Science, 2003;302:276-8.
8. Tennant BJ, Gaskell RM, Kelly DF, Carter SD, Gaskell CJ. Canine coronavirus infection in the dog following oronasal inoculation. Res Vet Sci. 1991;51:11-8.
9. Decaro N, Martella V, Ricci D, Elia G, Desario C, Campolo M, et al. Genotype-specific fluorogenic RT-PCR assays for the detection and quantitation of canine coronavirus type I and type II RNA in faecal samples of dogs. J Virol Methods, in press.
10. Horsburgh BC, Brierley I, Brown TD. Analysis of a 9.6 kb sequence from the 3' end of canine coronavirus genomic RNA. J Gen Virol. 1992;73:2849-62.
11. Sanchez-Morgado JM, Poynter S, Morris TH. Molecular characterization of a virulent canine coronavirus BGF strain. Virus Res. 2004;104:27-31.
12. Pratelli A, Decaro N, Tinelli A, Martella V, Elia G, Tempesta M, et al. Two genotypes of canine coronavirus simultaneously detected in fecal samples of dogs with diarrhea. J Clin Microbiol. 2004;42:1797-9.
13. Jonassen CM, Kofstad T, Larsen IL, Lovland A, Handeland K, Follestad A, et al. Molecular identification and characterization of novel coronaviruses infecting graylag geese (Anser anser), feral pigeons (Columbia livia) and mallards (Anas platyrhynchos). J Gen Virol. 2005;86:1597-607.

## Claims

1. An isolated canine coronavirus, wherein said virus is a pantropic variant of CCoV type II, and can be isolated from the lungs, spleen, liver, kidneys and brain of a naturally infected dog.

2. An isolated canine coronavirus according to claim 1, wherein the virus has as a part of its genome a polynucleotide sequence coding for the spike protein (S) (SEQ ID NO: 2), the non-structural proteins 3a, 3b, 3c, 7a and 7b (SEQ ID NO: 3, 4, 5, 6 and 7, respectively), the envelope protein E (SEQ ID NO: 8), the membrane protein M (SEQ ID NO: 9) or the nucleocapsid protein N (SEQ ID NO: 10).

3. An isolated canine coronavirus according to claim 2, containing the polynucleotide sequences coding for all said proteins.

4. An isolated canine coronavirus according to claims 1-3, wherein said virus has as a part of its genome the sequence represented by SEQ ID NO:1.

5. A coronavirus according to claim 1-4, which is inactivated or attenuated.

6. A vaccine composition containing, as an immunogenic component, an attenuated or inactivated coronavirus according to claim 5, together with pharmaceutically acceptable vehicles and excipients.

7. The vaccine composition according to claim 6, for use in the prophylaxis or treatment of infections caused by pantropic virulent CCoV in dogs.
